# EUROPÄISCHE PATENTSCHRIFT

(11) **EP 1 402 873 B1**
(45) Veröffentlichungstag und Bekanntmachung des Hinweises auf die Patenterteilung: **07.03.2007**
(21) Anmeldenummer: 03020305.3
(22) Anmeldetag: 09.09.2003
(51) Int. Cl.: A61K 6/10

(54) **Zweikomponentige Zubereitung**
Two-component prepraration
Préparation à deux composants

(30) Priorität: 24.09.2002 DE 10244693
(43) Veröffentlichungstag der Anmeldung: 31.03.2004
(73) Patentinhaber: Heraeus Kulzer GmbH, 63450 Hanau (DE)
(72) Erfinder: Schaub, Matthias, Dr., 40593 Düsseldorf (DE); Nehren, Klaus, 41539 Dormagen (DE); Freckmann, Michael, 50769 Köln (DE); Urbas, Holger, 47829 Krefeld (DE)
(74) Vertreter: Kühn, Hans-Christian

(56) Entgegenhaltungen:
- EP-A- 0 269 819
- EP-A- 0 901 785
- EP-A- 1 081 191
- EP-A- 1 226 808
- WO-A-01/79328
- DE-A- 4 439 769

## Beschreibung

Die vorliegende Erfindung betrifft zweikomponentige Zubereitungen, insbesondere zur dentalen Abformung, sowie deren Verwendung.

Unterschiedliche Arten an Zubereitungen zur dentalen Abformung sind an sich bekannt (siehe R.G. Craig, Restaurative Dental Materials, The C.V. Moosbe-Comp. St. Louis, Toronto, London, 1980, S. 1979 ff). An derartige Materialien werden insgesamt sehr hohe Anforderungen gestellt (vgl. K. Eichner, Zahnärztliche Werkstoffe und ihre Verarbeitung, Bd. 1, A. Hüthig Verlag, Heidelberg, 4. Auflage, 1981, S. 45 ff):
1. Angenehmer Geruch, Geschmack und ästhetisches Aussehen.
2. Die Massen dürfen keine toxischen oder irritierenden Bestandteile enthalten.
3. Die Massen müssen eine mehrmonatige Lagerstabilität aufweisen.
4. Die Massen müssen wirtschaftlich herstellbar sein und eine präzise Abformung ergeben.
5. Die Massen müssen leicht zu handhaben sein.
6. Die Härtungscharakteristik muss den klinischen Erfordernissen entsprechen.
7. Die ausgehärteten Massen müssen elastisch sein und dürfen sich nicht unter Zugbeanspruchung bleibend verformen.
8. Die ausgehärteten Massen müssen eine ausreichende Druckfestigkeit besitzen und dürfen nicht brechen.
9. Die ausgehärteten Massen müssen bei Raumtemperatur und normaler Luftfeuchtigkeit solange dimensionsstabil sein, dass in angemessener Zeit exakte Gipsabdrücke hergestellt werden können.
10. Die ausgehärteten Massen dürfen keine Gipsschädigung hervorrufen und müssen mit anderen Abformmassen kompatibel sein.

Aus der Gruppe der verschiedenen Materialien sind elastomere Abformmaterialien besonders vorteilhaft, u. a. auch aufgrund der gegenüber den nicht elastomeren Abformmaterialien vorteilhaften anwendungstechnischen und mechanischen Eigenschaften.

In der Regel liegen diese elastomeren Abformmaterialien vor der "Abbindung" (d. h., Formung der elastomeren Struktur) als Pasten vor, die in der Regel aus zwei Komponenten (häufig als Basispaste und Katalysator- oder Härterpaste bezeichnet) bestehen und zu dem Elastomer nach Vermischen abbinden (vernetzen).

Es sind verschiedene Arten von elastomeren Abformmaterialien bekannt, wie z. B. Elastomere mit Polysiloxankettenstruktur, die durch Kondensationsreaktion von hydroxyfunktionellen Polysiloxanen mit Alkoxy silan-Vemetzern abbinden (sogenannte C-Silicone) oder additionsvernetzende Polysiloxane (sogenannte A-Silicone), die durch eine Hydrosilierungsreaktion von Vinylgruppen an einem polydiorganylgruppenhaltigen Polymer (Vinylpolymere) mit einem SiH-Gruppen enthaltenden Polydiorganosiloxan (SiH-Vernetzer) miteinander reagieren und dadurch ein Elastomer formen.

Elastomere Abformmassen auf Basis von Polyetherderivaten sind im Dentalbereich ebenfalls seit langem bekannt, wie z.B. die häufig verwendeten Aziridinopolyether (z. B. in DE-B-17 45 810 beschrieben) oder additionsvernetzende Polyethermaterialien, wie sie z. B. in DE-A1-37 41 575 bzw. DE-A1-3838587 beschrieben sind. Polyetherabformmaterialien mit Acrylat- bzw. Methacrylatgruppen, sind z. B. aus EP 0 173 085 bekannt.

WO 0179328 A1 beschreibt 2K-Massen mit antacidem Zusatz auf Basis von Aziridinopolyethern. Gemäß Seite 18 kann man durch Variation der Mengenverhältnisse Säuren / antacide Verbindungen / Wasser gewünschte Verarbeitungszeiten einstellen.

Dentale Abformmassen auf Basis silanfunktionalisierter Polyetherderivate sind ebenfalls bekannt. EP 0 269 819 B1 beschreibt die Verwendung von Abmischungen enthaltend Ether-, Urethan- und Harnstoffgruppen enthaltende Polyadditionsprodukte mit Alkoxysilanendgruppen zur Herstellung von Abform- oder Dubliermassen im Dentalbereich.

In DE 43 08 024 und DE 44 39 769 werden sehr ähnliche Systeme offenbart, nämlich Kunststoffe mit mindestens einem Silan-, Ether- und Urethangruppen und ggf. Harnstoffgruppen enthaltenden Polyadditionsprodukt mit einer überwiegend linearen Molekülstruktur und überwiegend aliphatisch oder cycloaliphatisch gebundenen Ether oder Urethansegmenten und einem Zahlenmittel der Molmasse im Bereich von 800-20000, mit einem Gehalt an endständig angeordneten Silylgruppen, wobei mindestens eine Ether-Gruppe in mindestens einem der Substituenten am Siliziumatom vorliegt.

In DE 101 04 079.2-42 werden schließlich Mischungen auf Basis alkoxysilylfunktioneller Polyether mit linearer oder verzweigter Hauptkette als Abform- und Dubliermassen im Dentalbereich beschrieben. Darüber hinaus sind silanfunktionalisierte Polyetherderivate auch als Additive für die Aktivatorkomponenten von kondensationsvernetzende Silikonmassen bekannt. Derartige Systeme sind in DE 198 08 557 beschrieben.

Abformmassen auf Basis silanfuriktionalisierter Polyetherderivate nach dem Stand der Technik enthalten in der Katalysatokomponente neben Verdünnern, Füllstoffen und weiteren Modifikatoren als katalytisch aktive Komponenten acide Verbindungen und Wasser. Bei Abmischung von Basis und Katalysator erfolgt durch säurekatalysierte Hydrolyse- und Kondensationreaktionen an den Silanendgruppen eine Vernetzung und damit der Übergang in den elastomeren Zustand. Durch den Gehalt an acider Verbindung und an Wasser in der Katalysatorkomponente lässt sich die Härtungscharakteristik an die klinischen Erfordernisse anpassen.

Für den Anwender sind dabei die Verarbeitungszeit, also der Zeitraum zwischen Mischende und beginnender Vernetzung (Übergang der Mischung von der plastischen in die elastische Phase, gekennzeichnet durch stark verminderte Fließfähigkeit, Fädenziehen) und die Abbindezeit (Zeitraum zwischen Mischende und Weiterverarbeitbarkeit der aushärtenden Masse z. B. durch Mundentnahme) der Abformmasse wichtige Kenngrößen. In der Regel werden Basis- und Katalysatorkomponente so aufeinander abgestimmt, dass Verarbeitungszeiten im Bereich von 30s bis 3 min eingestellt werden. Die Abbindezeiten liegen in der Regel bei maximal 7 min.

Praxistaugliche Abformmassen müssen eine mehrmonatige Lagerstabilität aufweisen, d.h. physikalische Eigenschaften, wie z.B. Viskosität, Verarbeitungs- und Abbindezeit dürfen sich in diesem Zeitraum nicht wesentlich ändern. Wünschenswert sind Lagerstabilitäten über einen Zeitraum von 2 bis 3 Jahren.

Die Abformmassen können bei Lagerung und Transport auch zeitweise erhöhten Temperaturen ausgesetzt sein. Erhöhte Temperaturen reduzieren in der Regel die Lagerstabilität. Erfahrungsgemäß sollten Abformmassen bei einer Lagertemperatur von 60°C zumindest für eine Woche stabil bzw. verarbeitbar bleiben.

Die silanfunktionalisierte Polyetherderivate enthaltenden Basiskomponenten nach dem Stand der Technik weisen allerdings aufgrund ihrer Empfindlichkeit gegenüber Feuchte und gegenüber aciden Bedingungen den Nachteil eines deutlichen Viskositätsanstiegs bei Lagerung auf. Lagerung bei Raumtemperatur bewirkt innerhalb weniger Monate etwa eine Verdoppelung der Ausgangsviskosität. Dieser Effekt tritt bei erhöhten Temperaturen beschleunigt auf. Lagerung bei 60°C führt in der Regel nach einer Woche zu sehr hochviskosen oder vernetzten Produkten, die nicht mehr verarbeitbar sind.

Es besteht daher ein Bedarf an Zubereitungen auf Basis silanfunktionalisierter Polyetherderivate, die einerseits nach Abmischung mit aciden Katalysatoren ein praxistaugliches Abbindeverhalten aufweisen (d.h. Verarbeitungszeiten von 0,5 bis 3,5 min bei Raumtemperatur und Abbindezeiten, nach denen eine Mundentnahme möglich ist, von max. 7 min nach Mischbeginn) und andererseits als Einzelkomponente eine gegenüber dem Stand der Technik deutlich verlängerte Lagerstabilität bzw. einen nur geringfügigen Viskositätsanstieg bei zunehmender Lagerdauer aufweisen.

Aus dem Bereich der feuchtehärtenden Klebe- und Dichtungsmassen sind Systeme bekannt, die ebenfalls auf Basis silanfunktionalisierter Polyetherderivate formuliert werden (z.B. DE 36 29 237). Bei diesen Systemen, besteht in analoger Weise wie bei dentalen Abformmassen die Anforderung nach einer ausreichend langen Haltbarkeitsdauer. Daher ist es häufig sinnvoll, derartige Zubereitungen z.B. gegen eindringende Feuchtigkeit zu stabilisieren, um die Haltbarkeitsdauer (shelf-life) zu erhöhen. Eine solche Verbesserung der Haltbarkeit kann , wie z.B. in WO99/48942 beschrieben, durch den Einsatz von Feuchtigkeits-Stabilisatoren erreicht werden. Demnach eignen sich als Feuchtigkeitsstabilisatoren alle Verbindungen, die mit Wasser unter Bildung einer gegenüber den in den Zubereitung vorliegenden reaktiven Gruppe inerten Gruppe reagieren und hierbei möglichst geringe Veränderungen ihres Molekulargewichts eingehen. Weiterhin muss die Reaktivität der Stabilisatoren gegenüber in die Zubereitung eingedrungener Feuchtigkeit höher sein, als die Reaktivität der Endgruppen des in der Zubereitung vorliegenden silanfunktionalisierten Polyetherderivats. Als Feuchtigkeitsstabilisatoren eignen sich beispielsweise Isocyanate oder Silane, wie z.B. Vinylsilane, Oximsilane oder Benzamidosilane.

Aus dem Bereich der feuchtehärtenden Klebe- und Dichtungsmassen auf Basis silanfunktionalisierter Polyetherderivate ist weiterhin der Einsatz weiterer Zusatzstoffe, wie z. B. Amine, bekannt. Amine werden dabei als Katalysatoren zur Beschleunigung der Härtungsgeschwindigkeit (s. WO 99/48942, US 6,310,170) eingesetzt.

Spezielle sterisch gehinderte Amine werden z. B. im Konzentrationsbereich bis 2% als UV-Stabilisatoren (sogenannte Hindered Amine Light Stabilisators bzw. HALS) eingesetzt.

Für dentale Abformmassen und feuchtehärtende Klebe- und Dichtungsmassen auf Basis silanfunktionalisierter Polyetherderivate ist die Verwendung von verschiedenen Füllstoffen bekannt, wie z.B. Quarzmehl, Cristobalitmehl, Calciumsulfat, Diatomeenerde, Silikate, pyrogenes oder gefälltes Siliciumdioxid, Kreide, Kalkmehl, Zeolithe, Bentonite, Glaskugeln Glasmehl, Glasfasem und Gtasfasemkurzschnitte, Ruß.

Zur Formulierung von zweikomponentigen säurehärtenden Zubereitungen auf Basis Silanfunktionalisierter Polyetherderivate nach dem Stand der Technik werden in der Regel keine antacid wirkenden Füllstoffe oder Additive, wie Kreide, Kalkmehl, Zeolithe, alkalisch reagierende Silikate oder Diatomeenerde eingesetzt, um die Gefahr einer Neutralisation der sauren Katalysatorkomponente und damit eine verzögerte Aushärtung zu vermeiden.

Der Erfindung liegt die Aufgabe zugrunde, eine gegenüber dem Stand der Technik verbesserte Zubereitung zur Verfügung zu stellen.

Die Aufgabe wird erfindungsgemäß durch eine zweikomponentige, bei Raumtemperatur zu einem elastomeren Material abbindende Zubereitung gelöst, bestehend aus einer Basis-Komponente (A) und einer Säureenthaltenden Katalysator-Komponente (B), wobei

A mindestens ein silanfunktionalisiertes Polyetherderivat, mindestens eine antacidwirkende Verbindung und gegebenenfalls weitere Zusätze enthält.

In einer bevorzugten Ausführungsform enthält A 5-95% mindestens des silanfunktionalisierten Polyetherderivates, 0,002 - 15 % der mindestens einen antacidwirkenden Verbindung, 0 - 90% eines inerten Verdünnungsmittels, 0 - 60% weiterer Modifikatoren.

Die Erfindung betrifft auch die Verwendung der beschriebenen zweikomponentigen, bei Raumtemperatur zu einem elastomeren Material abbindenden Zubereitung zur Herstellung einen dentalen Abformung.

Überraschend wurde gefunden, dass Zubereitungen auf Basis silanfunkuonailsierter Polyetherderivate, die in der Basiskomponente (Komponente A) mindestens eine antacid wirkende Verbindunge und gegebenenfalls weitere Zusätze enthalten, deutlich verlängerte Haltbarkeitsdauern aufweisen und dabei gleichzeitig praxistaugliche physikalische Eigenschaften, insbesondere auch zum Abbindeverhalten aufweisen können. Die Erfindung ist gerichtet auf eine zweikomponentige Zusammensetzung auf der Basis silanfunktionalisierter Polyetherderivate, die nach Mischung von einer silanfunktionalisierte Polyetherderivate enthaltenden Basis-Komponente (Komponente A) mit einer sauren Katalysatorkomponente (Komponente B) bei Raumtemperatur zu einem elastomeren Produkt aushärten.

Insbesondere betrifft die Erfindung solche Zubereitungen auf Basis silan-funktionalisierter Polyetherderivate, die sich nach Mischung mit der Katalysatorkomponente durch anwendungstaugliche Verarbeitungszeiten und Abbindezeiten auszeichnen und dabei als Einzelkomponenten eine lange Haltbarkeitsdauer aufweisen.

Überraschend konnte festgestellt werden, dass Zubereitungen die antacidwirkende wirkende Verbindungen in den Konzentrationsbereichen enthalten, die zur Verlängerung der Haltbarkeitsdauer geeignet sind, nach Abmischung mit Katalysatorkomponenten noch praxistaugliche Verarbeitungs- und Abbindezeiten aufweisen können.

Durch Anpassung der Säurekonzentration der Katalysatorkomponente lässt sich die antacidwirkende wirkende Komponente der Basis-Paste in gewissen Konzentrationsbereichen so ausgleichen, dass die anwendungstechnisch gewünschte Verarbeitungs- und Abbindezeit eingestellt werden kann. Umgekehrt lässt sich ebenso die Konzentration der antacidwirkenden wirkenden Komponente in einem gewissen Rahmen so auf die Säurekomponente abstimmen, dass die anwendungstechnisch gewünschte Verarbeitungs- und Abbindezeit eingestellt wird.

Die antacid wirkenden Verbindung(en) weisen dabei einen Anteil von 0,0001 - 60 %, vorzugsweise von 0,002 - 15 % auf.

Weiterhin konnte überraschend festgestellt werden, dass durch Zusatz von speziellen antacidwirkenden wirkenden Verbindungen, wie z. B. Aminen, die nach dem Stand der Technik zur Beschleunigung der Härtungsgeschwindigkeit von Zubereitungen mit silanfunktionalisierten Polyetherderivaten eingesetzt werden, eine Verlängerung der Lagerstabilität (durch Verminderung des mit zunehmender Lagerdauer beobachteten Viskositätsanstiegs) erreicht werden kann. Neben Aminen bewirken viele andere antacidwirkende-wirkende Verbindungen, wenn sie Additive zur Basis-Komponente eingesetzt werden ebenfalls eine Verlängerung der Lagerstabilität.

Die antacid wirkenden Verbindungen werden bevorzugt aus den folgenden Gruppen ausgewählt: Basische oder amphotere Oxide, Hydroxide, Carbonate, Carboxylate, basische organische Verbindungen mit N, As, O, P, S oder Sb als Heteroatom.

Ebenfalls bevorzugt werden organische Verbindungen mit Isocyanat-, Epoxid-, Carbodiimid- oder Aziridino-Gruppen als antacid wirkende Verbindungen eingesetzt.

Weiterhin ist es bevorzugt, wenn stickstoffhaltige organische Verbindungen als antacidwirkende Verbindungen eingesetzt werden.

Die erfindungsgemäßen Zubereitungen auf Basis silanfunktionalisierter Polyetherderivate, die zur Abformung z. B. im Dentalbereich eingesetzt werden können, werden in der Regel als zweikomponentige Systeme, bestehend aus einer Komponente A und einer Komponente B formuliert.

Komponente A enthält als wesentliche Komponente zum Elastomeraufbau ein silanfunktionalisiertes Polyetherderivat oder eine Mischung mehrerer bzgl. Molekulargewicht und/oder chemischer Struktur unterschiedlicher silanfunktionalisierter Polyetherderivate.

Geeignete silanfunktionalisierte Polyetherderivate sind Polymere, die sich durch die Kombination des Strukturmerkmals der Polyetherkette mit dem Strukturmerkmal einer reaktiven Silan-End- und/oder Silan-Seitengruppe auszeichnen. Diese reaktiven Silan-End- und/oder Silan-Seitengruppen sind gekennzeichnet durch hydrolysierbare Gruppen und/oder Hydroxylgruppen am Si-Atom.

Geeignete silanfunktionalisierte Polyetherderivate können beispielsweise reine Polyether sein, die endständig mit Alkoxysilylresten funktionalisiert sind, wie z.B. die als "modified-silane polymers" (MS Polymer^{®}) bekannten Polyetherderivate.

Besonders geeignete silanfunktionalisierte Polyetherderivate sind silanterminierte Polyurethane und die aus EP 0 269 819 B1, bekannten Ether-, Urethan-, und Harnstoffgruppen enthaltenden silanfunktionalisierten Polyadditionsprodukte oder die aus DE 101 04 079.2-42 bekannten alkoxysilylfunktionellen Polyether mit linearer oder verzweigter Hauptkette.

Geeignete silanfunktionalisierte Polyetherderivate können aber auch auf Copolymeren von Polyethern mit anderen Polymeren, wie z.B. Polyestern, Polyolefinen oder Polyorganosiloxanen basieren

Das silanfunktionalisierte Polyetherderivat enthält somit vorzugsweise Urethangruppen oder Harnstoffgruppen oder Trialkoxysilyl- oder Alkyldialkoxysilylgruppen.

Die Komponente A der erfindungsgemäßen Zubereitungen enthält neben dem silanfunktionalisierten Polyetherderivat eine antacidwirkende Komponente oder eine Mischung mehrerer antacidwirkender Komponenten. Geeignete antacidwirkende Verbindungen sind generell anorganische oder organsiche Verbindungen, die Säuren neutralisieren können, wie z.B. Oxide, Hydroxide, Carbonate, Carboxylate bestimmter Metalle mit basischem oder amphoteren Charakter sowie basische organische Verbindungen mit N, As, O, P, S oder Sb als Heteroatom. Von den basischen organischen Verbindungen werden Amine und stickstoffhaltige Heterocyclen, wie z.B. Alkaloide, besonders bevorzugt als antacide Komponente eingesetzt. Schließlich können die Silan-funktionalisierten Polyetherderivate selbst, bei geeigneter Funktionalisierung z.B. mit Aminogruppen, als antacide Komponente wirken.

Weiterhin sind antacid wirkende Verbindungen im Sinne dieser Erfindung solche Verbindungen, die mit Säuren eine Additionsreaktion eingehen, wie z.B. Isocyanate, Carbodiimide, Epoxide oder Aziridine.

Als antacid wirkende Komponenten sind auch anorganische Füllstoffe geeignet, die mit antacid Wirkenden organischen Verbindungen oberflächenfunktionalisiert sind. Besonders bevorzugt sind dabei Füllstoffe, die mit welche Amino-, Isocyanat-, Epoxid-, Carbodiimid- oder Aziridino-Gruppen beladen sind.

Bevorzugt wird die antacidwirkende Verbindung in der Komponente A in einem Konzentrationsbereich von 0,0001 - 60 % eingesetzt. Besonders bevorzugt sind Konzentrationen von 0,002 - 15%.

Sowohl die Komponente A als auch die Komponente B können weitere übliche Modifikatoren enthalten, wie z. B. Verdünner, Weichmacher, Füllstoffe, Farbstoffe, Pigmente, Geruchs- und Geschmacksstoffe, Thixotropiermittel, Emulgatoren, Stabilisatoren.

Die Katalysator-Komponente (Komponente B) enthält als katalytisch aktive Verbindungen eine organische und/oder anorganische Säure bzw. Abmischungen verschiedener organischer und/oder anorganischer Säuren sowie Wasser. Bevorzugt werden Sulfonsäuren eingesetzt, besonders bevorzugt 4-Toluolsulfonsäure. Ebenfalls bevorzugt ist es , wenn die Katalysator-Komponente (B) Wasser enthält. Die Verwendung von mehreren Säuren kann zur Einstellung des Abbindeverlaufes hilfreich sein. Weiterhin kann die Verwendung bestimmter Salze, z.B. Fluoride wie Natriumfluorid, Kaliumfluorid oderAmmoniumfluorid sowie organischer Aminfluoride zur Einstellung des Härtungsverhaltens hilfreich sein.

Die Herstellung von Komponente A und Komponente B kann z. B. nach den in DE 101 04 079.2-42 beschriebenen Verfahren erfolgen.

Zur Verarbeitung können die Komponenten A und B im Verhältnis 20:1 bis 1:5 abgemischt werden. Vorzugsweise werden die Komponenten im Verhältnis 10:1 bis 1:1 abgemsicht.

Bevorzugt werden die Komponenten A und B in Tuben, Dosen, Schlauchbeuteln oder Doppelkartuschen dargeboten.

Nachfolgend werden Ausführungsbeispiele der Erfindung dargestellt. Basis-Komponeneten mit verschiedenen antacidwirkenden Verbindungen werden nach DE 101 04 079.2-42, Beispiel 5, hergestellt und anschließend in Alu-Tuben abgefüllt (Zusammensetzungen s. Tab. 1). Die Überprüfung der Lagerstabilität bei 60°C zeigt bei den erfindungsgemäßen Zubereitungen einen wesentlich geringeren Viskositätsanstieg gegenüber dem Stand der Technik (s. Tab. 2). Die Zubereitungen aus Tab. 1 lassen sich durch Abmischung mit Katalysatorkomponenten, die nach DE 101 04 079.2-42, Beispiel 3, hergestellt wurden (s. Tab. 3) innerhalb praxistauglicher Verarbeitungszeiten aushärten (s. Tab. 4).

**Tabelle 1: Zuaammensetzung der erfindungsgemäßen Basis-Komponenten (A1 - A6); Vergleichsbeispiel: A7**

| | | A1 | A2 | A3 | A4 | A5 | A6 | A7 |
|---|---|---|---|---|---|---|---|---|
| | | | | | | | | |
| | Silanterminiertes Polyetherderivat nach EP 0269 819 B1, Beispiel 3 | 20,00 | 20,00 | 20,00 | 20,00 | 20,00 | 20,00 | 20,00 |
| | Verdünner (Dibenzylzoluol) | 20,00 | 19,99 | 19,99 | 19,95 | 19,95 | 19,97 | 20,00 |
| | Füllstoff (Quarzmehl) | 56,80 | 57,00 | 58,50 | 58,50 | 57,00 | 57,00 | 58,50 |
| | Strukturmittel | 3,00 | 3,00 | 1,50 | 1,50 | 3,00 | 3,00 | 1,50 |
| | Antacidwirkende Verbindung: | | | | | | | |
| | Basisches Magnesium-Aluminium-Hydroxy-Carbonat, pH 9-10 | 0,20 | | | | | | |
| | 3-Aminopropyltriethoxysilan | | | 0,01 | 0, 5 | | | |
| | Polypropylenglykoldiamin, MW 230 | | | | | 0,05 | | |
| | 4-Hydroxy-2,2,6,6-Tetramethylpiperidin | | 0,01 | | | | | |
| | 1,8-Bis(dimethylamino)-naphthalin | | | | | | 0,03 | |

**Tabelle 2: Charakterisierung der Lagerstabilität der erfindungsgemäßen (A1-A6) Basis-Komponenten; Vergleichsbeispiel: A7**

| | | A1 | A2 | A3 | A4 | A5 | A6 | A7 |
|---|---|---|---|---|---|---|---|---|
| | Viskosität (23°C, 3s⁻¹) unmittelbar nach Herstellung [Pas] | 464 | 312 | 292 | 183 | 434 | 444 | 257 |
| | Viskosität (23°C, 3s⁻¹) nach einwöchiger Lagerung bei 60°C [Pas] | 1960 | 985 | 591 | 262 | 1167 | 2100 | nicht messbar, Material ausgehärtet |

**Tabelle 3: Säuregehalt verschiedener Katalysatorkomponenten (B1-B3), hergestellt nach DE 101 04 079.2-42, Beispiel 3**

| | | B1 | B2 | B3 |
|---|---|---|---|---|
| | Gew.-Anteil4-Toluolsulfonsäure-Monohydrat [%] | 0,27 | 0,31 | 0,51 |

**Tabelle 4: Verarbeitungszeiten- und Shore-Härten verschiedener Abmischungen aus Basis- und Katalysator-Komponenten; Beispiel 7: Vergleichsbeispiel**

| | Beispiel | 1 | 2 | 3 | 4 | 5 | 6 | 7 |
|---|---|---|---|---|---|---|---|---|
| | Verwendete Basis-Komponente | A1 | A2 | A3 | A4 | A5 | A6 | A7 |
| | Verwendete Katalysator-Komponente | B1 | B2 | B2 | B2 | B3 | B3 | B2 |
| | Gewichtsverhältnis von Basis- zu Katalysatorkomponente | 5:1 | 5:1 | 5:1 | 5,5:1 | 5:1 | 7:1 | 5:1 |
| | Verarbeitungszeit [min:s] | 2:30 | 2:55 | 2:35 | 2:30 | 3:40 | 2:55 | 2:15 |
| | Shore A (nach 60 min) | 55 | 56 | 58 | 55 | 57 | 58 | 59 |

## Patentansprüche

1. Zweikomponentige, bei Raumtemperatur zu einem elastomeren Material abbindende Zubereitung, bestehend aus einer Basis-Komponente (A) und einer Säureenthaltenden Katalysator-Komponente (B), wobei
A mindestens ein silanfunktionalisiertes Polyetherderivat,
mindestens eine antacidwirkende Verbindung
und gegebenenfalls weitere Zusätze enthält.

2. Zweikomponentige, bei Raumtemperatur zu einem elastomeren Material abbindende Zubereitung nach Anspruch 1, wobei die antacid wirkenden Verbindung einen Anteil von 0,0001 - 60 %, vorzugsweise von 0,002 - 15 %, aufweist.

3. Zweikomponentige, bei Raumtemperatur zu einem elastomeren Material abbindende Zubereitung nach Anspruch 1 oder 2, wobei
A 5-95% mindestens des silanfunktionalisierten Polyetherderivates,
0,002 - 15 % der mindestens einen antacidwirkenden Verbindung,
0-90% eines inerten Verdünnungsmittels,
0-60% weiterer Modifikatoren enthält.

4. Zweikomponentige, bei Raumtemperatur zu einem elastomeren Material abbindende Zubereitung nach einem der vorhergehenden Ansprüche, wobei das silanfunktionalisierte Polyetherderivat Urethangruppen enthält.

5. Zweikomponentige, bei Raumtemperatur zu einem elastomeren Material abbindende Zubereitung nach einem der vorhergehenden Ansprüche, wobei das silanfunktionalisierte Polyetherderivat Harnstoffgruppen enthält.

6. Zweikomponentige, bei Raumtemperatur zu einem elastomeren Material abbindende Zubereitung nach einem der vorhergehenden Ansprüche, wobei das silanfunktionalisierte Polyetherderivat Trialkoxysilyl- oder Alkyldialkoxysilylgruppen enthält.

7. Zweikomponentige, bei Raumtemperatur zu einem elastomeren Material abbindende Zubereitung nach einem der vorhergehenden Ansprüche, wobei die antacidwirkenden Verbindungen gewählt sind aus: Basische oder amphotere Oxide, Hydroxide, Carbonate, Carboxylate, basische organische Verbindungen mit N, As, O, P, S oder Sb als Heteroatom.

8. Zweikomponentige, bei Raumtemperatur zu einem elastomeren Material abbindende Zubereitung nach einem der vorhergehenden Ansprüche, wobei stickstoffhaltige organische Verbindunge als antacidwirkende Verbindungen eingesetzt werden.

9. Zweikomponentige, bei Raumtemperatur zu einem elastomeren Material abbindende Zubereitung nach einem der vorhergehenden Ansprüche, wobei organische Verbindungen mit Isocyanat-, Epoxid-, Carbodiimid- oder Aziridino-Gruppen als antacid wirkende Verbindungen eingesetzt werden.

10. Zweikomponentige, bei Raumtemperatur zu einem elastomeren Material abbindende Zubereitung nach einem der vorhergehenden Ansprüche, wobei die Katalysator-Komponente (B) 4-Toluolsulfonsäure enthält.

11. Zweikomponentige, bei Raumtemperatur zu einem elastomeren Material abbindende Zubereitung nach einem der vorhergehenden Ansprüche, wobei die Katalysator-Komponente (B) Wasser enthält.

12. Verwendung der zweikomponentigen, bei Raumtemperatur zu einem elastomeren Material abbindenden Zubereitung nach einem der vorhergehenden Ansprüche zur Herstellung eines dentalen Abformmaterials.

## Claims

1. Two-component preparation curing at room temperature to give an elastomeric material and consisting of a base component (A) and an acid-containing catalyst component (B), wherein
A contains at least one silane-functionalized polyether derivative,
at least one antacid compound
and optionally further additives.

2. Two-component preparation curing at room temperature to give an elastomeric material, according to claim 1, wherein the antacid compound has a proportion of 0.0001-60%, preferably of 0.002-15%.

3. Two-component preparation curing at room temperature to give an elastomeric material, according to claim 1 or 2, wherein
A contains 5-95% of the at least one silane-functionalized polyether derivative,
0.002-15% of the at least one antacid compound,
0-90% of an inert diluent,
0-60% of further modifiers.

4. Two-component preparation curing at room temperature to give an elastomeric material, according to any of the preceding claims, wherein the silane-functionalized polyether derivative contains urethane groups.

5. Two-component preparation curing at room temperature to give an elastomeric material, according to any of the preceding claims, wherein the silane-functionalized polyether derivative contains urea groups.

6. Two-component preparation curing at room temperature to give an elastomeric material, according to any of the preceding claims, wherein the silane-functionalized polyether derivative contains trialkoxysilyl or alkyldialkoxysilyl groups.

7. Two-component preparation curing at room temperature to give an elastomeric material, according to any of the preceding claims, wherein the antacid compounds are selected from basic or amphoteric oxides, hydroxides, carbonates, carboxylates, basic organic compounds having N, As, O, P, S or Sb as a heteroatom.

8. Two-component preparation curing at room temperature to give an elastomeric material, according to any of the preceding claims, wherein nitrogen-containing organic compounds are used as antacid compounds.

9. Two-component preparation curing at room temperature to give an elastomeric material, according to any of the preceding claims, wherein organic compounds having isocyanate, epoxide, carbodiimide or aziridino groups are used as antacid compounds.

10. Two-component preparation curing at room temperature to give an elastomeric material, according to any of the preceding claims, wherein the catalyst component (B) contains 4-toluenesulfonic acid.

11. Two-component preparation curing at room temperature to give an elastomeric material, according to any of the preceding claims, wherein the catalyst component (B) contains water.

12. Use of the two-component preparation curing at room temperature to give an elastomeric material, according to any of the preceding claims, for the preparation of a dental impression material.

## Revendications

1. Préparation biconstituante durcissant à température ambiante en un matériau élastomère constituée d'un constituant de base (A) et d'un constituant de catalyseur contenant un acide (B), dans laquelle
A contient au moins un dérivé de polyéther silane-fonctionnalisé,
au moins un composé ayant une action antiacide
et éventuellement d'autres additifs.

2. Préparation biconstituante durcissant à température ambiante en un matériau élastomère selon la revendication 1, dans laquelle le composé ayant une action antiacide présente une part de 0,0001-60 %, de préférence de 0,002-15 %.

3. Préparation biconstituante durcissant à température ambiante en un matériau élastomère selon la revendication 1 ou 2, dans laquelle
A contient au moins 5-95 % du dérivé de polyéther silane-fonctionnalisé,
0,002-15 % du au moins un composé ayant une action antiacide,
0-90 % d'un diluant inerte,
0-60 % d'autres agents de modification.

4. Préparation biconstituante durcissant à température ambiante en un matériau élastomère selon l'une quelconque des revendications précédentes, dans laquelle le dérivé de polyéther silane-fonctionnalisé contient des groupes uréthane.

5. Préparation biconstituante durcissant à température ambiante en un matériau élastomère selon l'une quelconque des revendications précédentes, dans laquelle le dérivé de polyéther silane-fonctionnalisé contient des groupes urée.

6. Préparation biconstituante durcissant à température ambiante en un matériau élastomère selon l'une quelconque des revendications précédentes, dans laquelle le dérivé de polyéther silane-fonctionnalisé contient des groupes trialcoxysilyle ou alkyldialcoxysilyle.

7. Préparation biconstituante durcissant à température ambiante en un matériau élastomère selon l'une quelconque des revendications précédentes, dans laquelle les composés ayant une action antiacide sont choisis parmi : des oxydes basiques ou amphotères, hydroxydes, carbonates, carboxylates, des composés organiques basiques avec N, As, O, P, S ou Sb comme hétéroatome.

8. Préparation biconstituante durcissant à température ambiante en un matériau élastomère selon l'une quelconque des revendications précédentes, dans laquelle on utilise des composés organiques contenant de l'azote comme composés ayant une action antiacide.

9. Préparation biconstituante durcissant à température ambiante en un matériau élastomère selon l'une quelconque des revendications précédentes, dans laquelle on utilise des composés organiques avec des groupes isocyanate, époxyde, carbodiimide ou aziridino comme composés ayant une action antiacide.

10. Préparation biconstituante durcissant à température ambiante en un matériau élastomère selon l'une quelconque des revendications précédentes, dans laquelle le constituant de catalyseur (B) contient de l'acide 4-toluènesulfonique.

11. Préparation biconstituante durcissant à température ambiante en un matériau élastomère selon l'une quelconque des revendications précédentes, dans laquelle le constituant de catalyseur (B) contient de l'eau.

12. Utilisation de la préparation biconstituante durcissant à température ambiante en un matériau élastomère selon l'une quelconque des revendications précédentes pour la préparation d'un matériau de moulage dentaire.
